# EUROPEAN PATENT SPECIFICATION

(11) **EP 0 259 820 B1**
(45) Date of publication and mention of the grant of the patent: **26.04.1995**
(21) Application number: 87113064.7
(22) Date of filing: 07.09.1987
(51) Int. Cl.: G01N 27/30, G01N 33/34

(54) **Procedure for controlling cellulose digestion**
Verfahren zur Regelung von Zellulosendigestion
Procédé de contrôle de la digestion de cellulose

(30) Priority: 08.09.1986 FI 863615
(43) Date of publication of application: 16.03.1988
(73) Proprietor: SAVCOR-CONSULTING OY, SV-50100 Mikkeli (FI)
(72) Inventor: Savisalo, Hannu, SF-50100 Mikkeli (FI); Kerola, Timo, SF-50500 Mikkeli (FI)
(74) Representative: Bühling, Gerhard, Dipl.-Chem.

(56) References cited:
- FR-A- 2 288 816
- FR-A- 2 526 950
- US-A- 4 104 028
- TAPPI JOURNAL vol. 66, no. 1, January 1983, pages 52-54; J.O. SOEDERBERG et al.: "The use of on-line alkali measurement in the computer control of a continuous digester"

## Description

The present invention concerns a procedure for controlling cellulose digestion by measuring the activity of the chemicals with essential effect on cellulose digestion which are present in the cellulose digester, the supplying of chemicals to be added into the cellulose digester being controlled on the basis of the result of measurement.

Information on various process variables is required in the control of cellulose digestion. The progress of the digesting process is also followed with the aid of the dissolving lignin, in addition to control of the incoming and outgoing pulp flow.

Determining the lignin content of the pulp itself would best reflect the progress of digestion, but the determination thereof is too time-consuming; and it is moreover exceedingly difficult to obtain a representative sample of the pulp during digestion. For these reasons, one has restricted the monitoring of digestion to analyzing the chemicals concentrations of the digesting solution. The variable on which attention is most commonly concentrated is the active, or effective, alkali.

The commonest analytic method for determining the effective alkali is based on titration.

Perhaps best known among computer-controlled analyzers of effective alkali in continuous action digestion is the apparatus in which titration is carried out with carbon dioxide. Titration is effected under pressure by measuring the reaction temperature and determining the end-point of titration from the titration graph with the aid of a computer.

Apparatus based on calorimetric titration has also been elaborated for analysis of effective alkali.

The drawback encumbering these methods is that in the sampling process samples cannot be taken at arbitrary points in the cellulose digester and therefore the sample is not representative enough. Moreover, analysis is based on content, not on activity; this detracts from the reliability of results. The apparatus designs are expensive and complex and this implies high chances of error. Maintenance also involves considerable expense.

Conductivity measurement has been increasingly employed towards measuring effective alkali, this method being simple and inexpensive. On the other hand, several factors exert an influence on conductivity, such as temperature, sodium carbonate and sodium sulphide, and changes in them have to be taken into account.

However, the selectivity of conductivity measurement is not good enough because the measuring technique fails to react swiftly enough to ions. Problems, and errors in measurement, are furthermore caused by electrode contamination. Cleaning and recalibration and mensuration, which is necessary thereafter, increase the operating costs.

The use of an ion-selective electrode is based on the activity of the ion, which can be imagined as a kind of effective concentration. The potential which the electrode yields is a function of the logarithm of activity. Best known among ion-selective electrodes is the pH pick-up, its output value being a function of the logarithm of the hydrogen ion activity.

The advantages of a measuring method based on ion selectivity include the placeability of pick-ups in the process flow, whereby there is no need to provide for sampling, which is often quite cumbersome. With ion-selective electrodes used in conjunction with the sulphate process slightly better selectivity of information is achieved, compared with other analytic methods which are practiced, although this selectivity is not good enough. Far this application, sodium and sulphide electrodes are commercially available, in addition to the pH electrode.

All methods outlined in the foregoing present either the drawback that they require inconvenient sampling or the drawback that the results of analysis are not selective enough.

FR-A-2 526 950 describes a process for analyzing components which are present in bleaching solutions used in paper industries. This process comprises the potentiometric determination of analytes by using a three-electrode system comprising a working electrode, a counter electrode and a reference electrode. On reaction current is supplied to the thus formed circuit from a current source in a manner such that a predetermined voltage between the measuring electrode and the reference electrode is maintained constant and measuring that current flowing between the measuring electrode and the counter electrode while this voltage is kept substantially constant. That measured current is directly proportional to the concentration of the chemical compounds to be investigated and, thus, the content of these compounds can be monitored on the basis of the measuring results.
However, the method according to this reference the dissolution of the measuring electrode has to be avoided, that is, the measuring electrode is made of an inert precious metal such as platinum.

The object of the invention is to provide a procedure in which the drawbacks associated with procedures of prior art have been avoided.

Therefore, and in order to accomplish those aims which will become apparent later on, the invention is mainly characterized in that the measurement is performed by placing in the cellulose digester one or several measuring electrodes and reference electrodes, and a current-supplying counterelectrode, electric current being supplied from a current source into the circuit thus established, in such manner that the voltage across the measuring electrode and the reference electrode, in other words the electrochemical potential of the measuring electrode, is substantially constant during the measurement, in which case the current intensity corresponding to the respective potential is directly proportional to the activity of the chemicals.

The invention makes use of the relationship between the current intensity of a dissolving electrode and the chemicals concentrations. That is, the present invention is directed to a
method for controlling cellulose digestion by measuring activity of chemicals which influence cellulose digestion and which are present in a cellulose digester, comprising
placing in digesting liquor within the cellulose digester, at least one measuring electrode (12), at least one reference electrode (13), and a current-supplying counterelectrode (14) to establish a circuit,
while digestion is being carried out in said digester, supplying current to the thus-established circuit from a current source (15) in a manner such that a predetermined voltage (U) between the measuring electrode (12) and the reference electrode (13), which represents electrochemical potential of the measuring electrode (12), is obtained,
maintaining the voltage (U) between the measuring electrode (12) and the reference electrode (13) substantially constant and measuring current flowing between the measuring electrode (12) and counterelectrode (14) in said digester while said voltage (U) is substantially constant, which is current intensity (I) corresponding to said electrochemical potential and is directly proportional to the cellulose digestive activity of said chemicals whereby the cellulose digestive activity is continuously determined directly in the cellulose digester, and
feeding ingredients into the digesting liquor within the cellulose digester based on said measuring results of current intensity, so that the feeding of ingredients into the cellulose digester can be controlled on the basis of the measuring results, said method being characterised in that the voltage (U) between the measuring electrode (12) and the reference electrode (13) is maintained substantially constant in a range at which dissolution (oxidation) of the measuring electrode (12) occurs.

In the measurement, a metallic surface is used which has with a suitable electric current been transformed into active state so that it will best react to changes in the desired ion concentration. The alkali content and sulphidity of the digesting liquor and their changes are most clearly and rapidly discernible in the current densities of an appropriate dissolving metal.

The procedure of the invention affords the further advantage over the state of art that one may without apprehension install a plurality of measuring pick-ups in various parts of the digesting vessel, whereby the sample will be representative. The measuring electrodes consist of metal and are therefore mechanically durable. Since a dissolving electrode is employed for measurement, contamination of the pick-up causes no problem. By the procedure, data are rapidly and accurately obtained concerning the chemicals concentrations in cellulose digesting in a manner enabling them to be used to control the chemical inputs.

In the procedure of the invention, a measuring pick-up has been installed in the cellulose digester with the aid of wall-traversing inlets, this pick-up comprising a metallic measuring electrode, a current-supplying counterelectrode and a reference electrode measuring electrochemical potential. The test body and counterelectrode of the measuring pick-up form a circuit which is supplied with electric current from a current source in such manner that the present voltage across the reference electrode and the test body is held constant. With this arrangement, the current intensity in the circuit is dependent on the chemicals concentration in the digesting liquor.

The quantity of effective alkali is directly proportional to the current intensity in the measuring circuit. The current passes between the measuring electrode and the counterelectrode when in the potentiostatic circuit the differential voltage between measuring electrode and counterelectrode has been adjusted to have a suitable magnitude.

In determination both of alkalinity and of sulphidity, the suitable voltage is in the range from -500 to -1500 mV with reference to a calomel electrode.

The measuring electrode may consist of carbon steel, iron, copper, zinc, cadmium or monel metal. The reference electrode may be any electrode which is compatible with the system.

The invention is described in detail with reference being made to an advantageous embodiment of the invention, presented in the figures of the attached drawings, yet to which the invention is not meant to be exclusively confined.

Fig. 1 presents a pick-up design employed in implementing the procedure of the invention, applied in a cellulose digester, in schematic elevational view.

Fig. 2 illustrates graphically the results of measurement obtained when using the procedure of the invention.

Fig. 3 illustrates graphically the current intensity/potential relationship of two different ions.

Fig. 4 presents the circuit used in the procedure of the invention, in a schematic diagram.

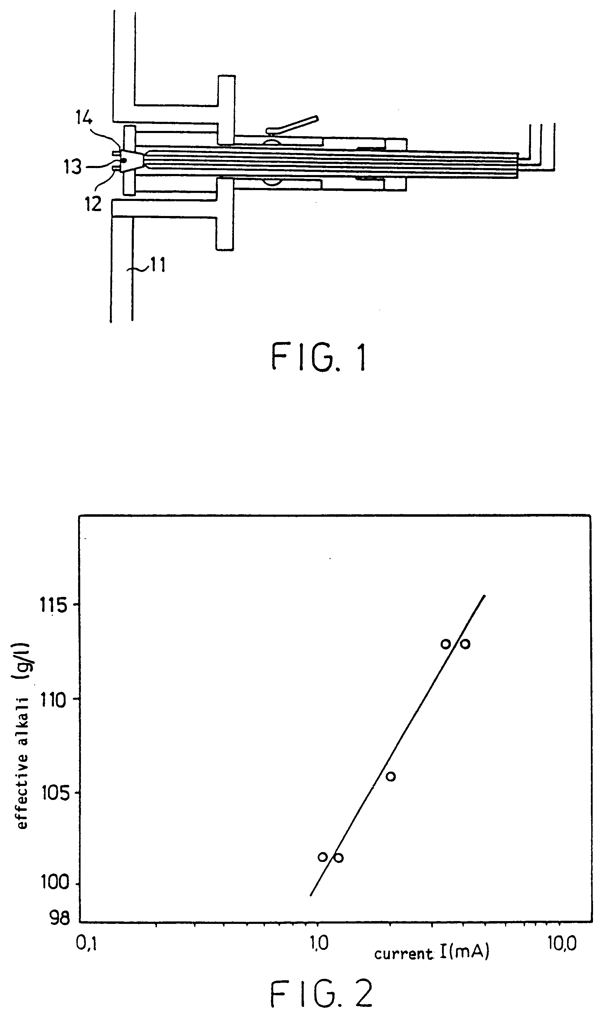

In Fig. 1 is depicted one way of introducing the pick-up in the cellulose digester. Obviously this may equally be arranged in other ways. Moreover, the circuit itself may be disposed in another way, without digressing from the inventive idea.

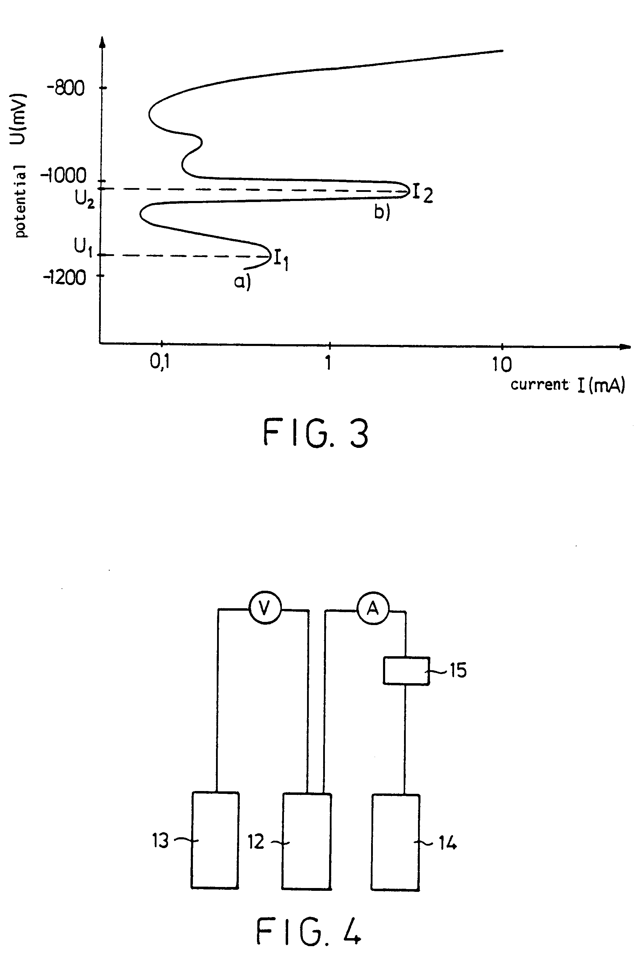

In the embodiment of Figs 1 and 4, the wall of the cellulose digester has been indicated with reference numeral 11, the measuring electrode with 12, the reference electrode with 13, and the counterelectrode with 14. In the circuit diagram of Fig. 4, the current source has been indicated with reference numeral 15.

In Fig. 2 are presented some results of measurements which have been obtained using the procedure of the invention. In Fig. 2 the y axis represents the quantity of effective alkali in units of g/l, and the x axis represents the current I flowing in the measuring circuit, in units of mA. The current intensity I has been found from the current flowing between the measuring electrode 12 and the counterelectrode 14 when in the potentiostatic circuit the differential voltage between the measuring electrode 12 and the reference electrode 13 has been adjusted to a suitable value.

The procedure of the invention is highly selective in respect of different ions. In Fig. 3 is presented a schematic diagram showing the current intensity/potential relationship for two different ions, (a) and (b). The most favourable situation obtains when U₁ corresponds to current intensity I₁ and U₂ to current intensity I₂.

## Claims

1. Method for controlling cellulose digestion by measuring activity of chemicals which influence cellulose digestion and which are present in a cellulose digester, comprising
placing in digesting liquor within the cellulose digester, at least one measuring electrode (12), at least one reference electrode (13), and a current-supplying counterelectrode (14) to establish a circuit,
while digestion is being carried out in said digester, supplying current to the thus-established circuit from a current source (15) in a manner such that a predetermined voltage (U) between the measuring electrode (12) and the reference electrode (13), which represents electrochemical potential of the measuring electrode (12), is obtained,
maintaining the voltage (U) between the measuring electrode (12) and the reference electrode (13) substantially constant and measuring current flowing between the measuring electrode (12) and counterelectrode (14) in said digester while said voltage (U) is substantially constant, which is current intensity (I) corresponding to said electrochemical potential and is directly proportional to the cellulose digestive activity of said chemicals whereby the cellulose digestive activity is continuously determined directly in the cellulose digester, and
feeding ingredients into the digesting liquor within the cellulose digester based on said measuring results of current intensity, so that the feeding of ingredients into the cellulose digester can be controlled on the basis of the measuring results, characterized in that
the voltage (U) between the measuring electrode (12) and the reference electrode (13) is maintained substantially constant in a range at which dissolution (oxidation) of the measuring electrode occurs.

2. Procedure according to claim 1, characterized in that for measuring electrode (12) is used carbon steel, iron, copper, zinc, cadmium or monel metal.

3. Procedure according to claim 1 or 2, characterized in that the electrochemical potential of the measuring electrode (12) is in the range from -500 to -1500 mV with reference to a calomel electrode.

## Patentansprüche

1. Verfahren zur Steuerung von Zellstoffkochung durch Messung der Aktivität von Chemikalien, die die Zellstoffkochung beeinflussen und in einem Zellstoffkocher anwesend sind, umfassend die folgenden Schritte:
mindestens eine Meßelektrode (12), mindestens eine Referenzelektrode (13) und eine stromzuführende Gegenelektrode (14) werden in eine Kochflüssigkeit in dem Zellstoffkocher gebracht, wobei eine Schaltung errichtet wird,
während die Kochung in dem Kocher durchgeführt wird, wird Strom der somit errichteten Schaltung von einer Stromquelle (15) in einer Weise zugeführt, so daß eine vorbestimmte Spannung (U) zwischen der Meßelektrode (12) und der Referenzelektrode (13), die das elektrochemische Potential der Meßelektrode (12) darstellt, erhalten wird,
die Spannung (U) Zwischen der Meßelektrode (12) und der Referenzelektrode (13) wird im wesentlichen konstant gehalten und der Strom, der zwischen der Meßelektrode (12) und der Gegenelektrode (14) in dem Kocher fließt, wird gemessen, während die Spannung (U) im wesentlichen konstant ist, wobei dies die Stromstärke (I) ist, die dem elektrochemischen Potential entspricht, und direkt proportional zu der Zellstoffkochaktivität der Chemikalien ist, wodurch die Zellstoffkochaktivität kontinuierlich direkt in dem Zellstoffkocher bestimmt wird, und
Bestandteile werden auf der Grundlage der Meßergebnisse der Stromstärke in die Kochflüssigkeit in dem Zellstoffkocher zugeführt, so daß die Zufuhr von Bestandteilen in den Zellstoffkocher auf der Grundlage der Meßergebnisse gesteuert werden kann, wobei das Verfahren dadurch gekennzeichnet ist, daß
die Spannung (U) zwischen der Meßelektrode (12) und der Referenzelektrode (13) im wesentlichen in einem Bereich konstant gehalten wird, in dem Auflösung (Oxidation) der Meßelektrode (12) auftritt.

2. Verfahren nach Anspruch 1, dadurch gekennzeichnet, daß für die Meßelektrode (12) Kohlenstoffstahl, Eisen, Kupfer, Zink, Cadmium oder Monelmetall verwendet wird.

3. Verfahren nach einem der Ansprüche 1 oder 2, dadurch gekennzeichnet, daß das elektrochemische Potential der Meßelektrode (12) in dem Bereich von -500 bis -1500 mV in Bezug auf eine Kalomel-Elektrode ist.

## Revendications

1. Procédé de régulation de la digestion de la cellulose en mesurant l'activité de produits chimiques qui influencent la digestion de la cellulose et qui sont présents dans un digesteur de cellulose, consistant :
à placer dans la liqueur de digestion dans le digesteur de cellulose au moins une électrode de mesure (12), au moins une électrode de référence (13) et une contre-électrode d'alimentation de courant (14) pour établir un circuit,
tandis que la digestion est mise en oeuvre dans ledit digesteur, à alimenter du courant au circuit ainsi établi à partir d'une source de courant (15) de telle forte qu'une tension prédéterminée (U) entre l'électrode de mesure (12) et l'électrode de référence (13) qui représente le potentiel électrochimique de l'électrode de mesure (12), soit obtenue,
à maintenir la tension (U) entre l'électrode de mesure (12) et l'électrode de référence (13) essentiellement constante et à mesurer le courant s'écoulant entre l'électrode de mesure (12) et la contre-électrode (14) dans ledit digesteur tandis que ladite tension (U) est essentiellement constante, qui est l'intensité de courant (I) correspondant audit potentiel électrochimique et est directement proportionnelle à l'activité digestive de la cellulose desdits produits chimiques si bien que l'activité digestive de la cellulose est déterminée directement en continu dans le digesteur de cellulose et
à alimenter les ingrédients dans la liqueur de digestion dans le digesteur de cellulose en fonction des résultats de ladite mesure de l'intensité de courant si bien que l'alimentation des ingrédients dans le digesteur de cellulose peut être régulée sur la base des résultats de mesure, caractérisé en ce que
la tension (U) entre l'électrode de mesure (12) et l'électrode de référence (13) est maintenue essentiellement constante dans une gamme dans laquelle la dissolution (oxydation) de l'électrode de mesure se produit.

2. Procédé selon la revendication 1, caractérisé en ce que pour l'électrode de mesure (12), on utilise de l'acier au carbone, du fer, du cuivre, du zinc, du cadmium ou du monel.

3. Procédé selon la revendication 1 ou 2, caractérisé en ce que le potentiel électrochimique de l'électrode de mesure (12) est compris dans la gamme de - 500 à - 1500 mV en référence à une électrode au calomel.
